# EUROPÄISCHE PATENTANMELDUNG

(11) **EP 4 353 806 A1**
(43) Veröffentlichungstag der Anmeldung: **17.04.2024**
(21) Anmeldenummer: 23191815.2
(22) Anmeldetag: 17.08.2023
(51) Int. Cl.: C11D 1/62, A61Q 5/02, C11D 1/66

(54) **TENSIDMISCHUNGEN**

(30) Priorität: 14.10.2022 DE 102022210879
(71) Anmelder: Henkel AG & Co. KGaA, 40589 Düsseldorf (DE)
(72) Erfinder: Birnbach, Janine, 51371 Leverkusen (DE); Schmiedel, Peter, 40591 Düsseldorf (DE)

(57) **Zusammenfassung**

Wasch- und reinigungsaktive Wirkstoffe sollten bereitgestellt werden, welche die Herstellung optisch ansprechender, konzentrierter fließfähiger Wasch-, Reinigungs- und Haarbehandlungsmittelzubereitungen ermöglichen und die in einfacher und effizienter Weise herstellbar sind, eine gute Lagerfähigkeit aufweisen und sich insbesondere durch gute Wasch- und Reinigungsergebnisse auszeichnen. Dies wurde durch eine Mischung aus einem Rhamnolipid und einer Verbindung der allgemeinen Formel (I)

X^{a-} a N⁺(R¹R²R³R⁴) (I)

in der
X^{a-} ein Anion ist,
a für 1, 2 oder 3 steht und
R¹ ausgewählt ist aus R² und H, R² ausgewählt wird aus C₁- bis C₄-Alkylresten und C₂- bis C₄-Hydroxyalkylresten, R³ ausgewählt ist aus R⁴ und R², und R⁴ ausgewählt ist aus C₁₀- bis C₁₈-Alkylresten, C₇- bis C₂₀-Alkylarylresten, Resten -R⁵-O(C=O)-R⁶ und Resten -R⁵-NH(C=O)-R⁶, wobei R⁵ aus den C₂- bis C₄-Alkylenresten und R⁶ aus den C₁₀- bis C₁₈-Alkylresten ausgewählt ist, und in R¹, R², R³, R⁴ und R⁶ jeweils die Alkylanteile linear oder verzweigtkettig sein können, erreicht.

## Beschreibung

Die vorliegende Erfindung betrifft Mischungen aus waschaktiven Verbindungen, die den Einzelkomponenten der Mischung überlegen sind. Weiterhin betrifft die Anmeldung Wasch-, Reinigungs- und Haarbehandlungsmittelmittel, welche diese Mischungen enthalten, sowie Wasch-, Reinigungs- und Haarbehandlungsverfahren unter Einsatz der Mischungen.

An die Konfektions- und Angebotsformen von Wasch-, Reinigungs- und Haarbehandlungsmitteln werden sich kontinuierlich ändernde Anforderungen gestellt. Ein für die Herstellung von derartigen Mitteln relevanter Trend ist deren Aufkonzentration. Hintergrund dieser Entwicklung sind neben einer höheren Verbraucherakzeptanz aufgrund vereinfachter Handhabung insbesondere Nachhaltigkeitsaspekte, beispielsweise in Bezug auf Transportvolumina und -kosten und die Menge der eingesetzten Verpackungsmittel.

Die Aufkonzentration moderner Wasch-, Reinigungs- und Haarbehandlungsmittel, insbesondere moderner Flüssigwaschmittel, beeinflusst in der Regel deren optische und rheologische Eigenschaften, hat Auswirkungen auf die Lagerstabilität dieser Mittel und kann deren Reinigungsleistung beeinflussen, insbesondere dann, wenn die hohe Konzentration der Aktivstoffe zu Unverträglichkeiten führt.

Darüber hinaus geht die Aufkonzentration flüssiger Mittel in der Regel mit dem Einsatz organischer Lösungsmittel einher. Diese Lösungsmittel verbessern die physikalische Stabilität der Wasch-, Reinigungs- und Haarbehandlungsmittel, zeichnen sich häufig jedoch nur durch eine geringe Wasch- oder Reinigungsaktivität aus, weshalb ihr Zusatz nicht zuletzt aus Nachhaltigkeitsgründen in geringstmöglichen Mengen erfolgen sollte. Der Einsatz von wässrigen oder wässrig-organischen Lösungsmittelsystemen bei der Herstellung aufkonzentrierter flüssiger Mittel ist mit den herkömmlichen wasch- oder reinigungsaktiven Wirkstoffen bisher jedoch nicht vermeidbar.

Mischungen von Aniontensiden mit Kationtensiden werden auch als Catanionics bezeichnet. Ihre Eigenschaften unterscheiden sich oft wesentlich von denen der Mischungeskomponenten; die Mischungen sind in der Regel lipophiler, weniger wasserlöslich und der Einfluss gleichzeitg anwesender Elektrolyte auf die physikalischen Eigenschaften der Mischungen ist normalerweise weniger stark ausgeprägt. Allerdings beobachtet man bei Anion-Kationtensidmischungen oft, dass die Mischungen präzipitierten und/oder flüssigkristalline oder gelförmige Phasen mit nicht optimalen Anwendungseigenschaften bildeten. Bekannt ist, dass die Zugabe von Alkoholen zur Lösung dieses Problems beiträgt, welches auch bei Mischungen von Tensiden mit unterschiedlichen Kettenlängen und gegebenfalls Verzweigungen weniger stark ausgeprägt sein kann. In diesen Fällen ist oft aber auch eine positive Wechselwirkung zwischen Aniontensid und Kationtensid weniger starkaugeprägt.

Aus der Patentschrift EP 0 499 434 B1 sind Waschmittel bekannt, die 1 bis 60 Gew.-% eines bei pH 7,0 und 25 °C in 1 gewichtsprozentigerwässriger Lösung eine micellare Phase ausbildendes Tensid und ein bei pH 7,0 und 25 °C in 1 gewichtsprozentiger wässriger Lösung eine lamellare Phase ausbildendes Tensid enthalten, wobei Rhamnolipid sowohl das die micellare Phase wie auch das die lamellare Phase ausbildende Tensid sein kann. Die europäische Patentschrift EP 1 445 302 B1 betrifft Waschmittel, die mindestens ein Glykolipid-Biotensid und mindestens ein Nicht-Glykolipid-Tensid enthalten, wobei diese sich in einer micellaren Phase befinden. Aus der internationalen Patentanmeldung WO 2012/010405 A1 sind Reinigungsmittel bekannt, die mindestens 1 Gew.-% Biotensid und ein Enzym bakterieller Herkunft enthalten. Die europäische Patentanmeldung EP 0 605 308 A1 offenbart Zusammensetzungen, die 0,001 Gew.-% bis 99,99 Gew.-% anionisches und/oder nichtionisches Tensid und 0,001 Gew.-% bis 99,99 Gew.-% Glykolipid enthalten, wobei unter den Glykolipiden zum Beispiel Sophorolipide, Rhamnolipide, Glucoselipide, Trehaloselipide und Cellobioselipide sein können. Aus der internationalen Patentanmeldung WO 2012/010406 A1 sind Waschmittel bekannt, die Lipase und Rhamnolipid enthalten, wobei das Rhamnolipid zu mindestens 50 Gew.-% aus Mono-Rhamnolipid besteht. Aus der internationalen Patentanmeldung WO 2012/010407 A1 sind Waschmittel bekannt, die Glykolipid-Tensid und Lipase bakterieller Herkunft enthalten, wobei das Glykolipid-Tensid zu mindestens 20 Gew.-% aus disaccharidischem säuregruppen-aufweisenden Glykolipid-Tensid besteht; das dort offenbarte Rhamnolipid besteht zu ca. 30 Gew.-% aus Mono-Rhamnolipid und zu ca. 70 Gew.-% aus Di-Rhamnolipid. Die europäische Patentanmeldung EP 2 410 039 A1 betrifft mono- und di-rhamnolipidhaltige Reinigungsmittel, in denen das Gewichtsverhältnis von Mono-Rhamnolipid zu Di-Rhamnolipid im Bereich von 95:5 bis 45:55 liegt; konkret offenbart werden Mittel, in denen das Gewichtsverhältnis von Mono-Rhamnolipid zu Di-Rhamnolipid bei 50:50 und zum Vergleich bei etwa 25:75 liegt. Die europäische Patentanmeldung EP 2 787 065 A1 betrifft mono- und di-rhamnolipidhaltige Textilwaschmittel, in denen das Gewichtsverhältnis von Di-Rhamnolipid zu Mono-Rhamnolipid größer als 51:49 ist; in der dort konkret offenbarten Rhamnolipidzusammensetzung aus P. putida liegt das Gewichtsverhältnis von Mono-Rhamnolipiden zu Di-Rhamnolipiden bei 1:99, wobei das Di-Rhamnolipid zu etwa 18 Gew.-% aus Di-Rhamnolipid mit mindestens einer C₁₂-Carbonsäure besteht. Aus der europäischen Patentanmeldung EP 2 786 743 A1 sind Rhamnolipidmischungen bekannt, die 51 Gew.-% bis 95 Gew.-% eines bestimmten Di-Rhamnolipids und 0,5 Gew.-% bis 9 Gew.-% eines bestimmten Mono-Rhamnolipids enthalten, in denen das Gewichtsverhältnis von Di-Rhamnolipid zu Mono-Rhamnolipid größer als 91:9 ist.

Der vorliegenden Erfindung lag die Aufgabe zugrunde, wasch- und reinigungsaktive Wirkstoffe bereitzustellen, welche die Herstellung optisch ansprechender, konzentrierter fließfähiger Wasch-, Reinigungs- und Haarbehandlungsmittelzubereitungen ermöglichen. Die Wirkstoffe sollten in einfacher und effizienter Weise herstellbar sein, eine gute Lagerfähigkeit aufweisen und sich insbesondere durch gute Wasch- und Reinigungsergebnisse auszeichnen.

Diese Aufgabe wurde durch eine Mischung tensidischer Wirkstoffe gelöst.

Ein erster Erfindungsgegenstand ist eine Mischung aus einem Rhamnolipid und einer Verbindung der allgemeinen Formel (I)

X^{a-} a N⁺(R¹R²R³R⁴) (I)

in der
X^{a-} ein Anion ist,
a für 1, 2 oder 3 steht und
R¹ ausgewählt ist aus R² und H, R² ausgewählt wird aus C₁- bis C₄-Alkylresten und C₂- bis C₄-Hydroxyalkylresten, R³ ausgewählt ist aus R⁴ und R², und R⁴ ausgewählt ist aus C₁₀- bis C₁₈-Alkylresten, C₇- bis C₂₀-Alkylarylresten, Resten -R⁵-O(C=O)-R⁶ und Resten -R⁵-NH(C=O)-R⁶, wobei R⁵ aus den C₂- bis C₄-Alkylenresten und R⁶ aus den C₁₀- bis C₁₈-Alkylresten ausgewählt ist, und in R¹, R², R³, R⁴ und R⁶ jeweils die Alkylanteile linear oder verzweigtkettig sein können.

Das Anion X^{a-} wird vorzugsweise ausgewählt aus Acetat, Formiat, Lactat, Oxalat, Chlorid, Bromid, lodid, Hydroxid, Sulfat, Hydrogensulfat, Carbonat, Hydrogencarbonat, Benzolsulfonat, Benzoat, Citrat, Mono- und Dihydrogencitrat, und deren Mischungen. Das Kation N⁺(R¹R²R³R⁴) ist entsprechend mehrfach vorhanden, wenn das Anion eine höhere Ladung als 1 aufweist (a in Formel I).

Vorzugsweise liegt das molare Verhältnis von Rhamnolipid : [Verbindung der allgemeinen Formel (I) : a] im Bereich von 100:1 bis 1:1, insbesondere von 10:1 bis 1:1 und besonders bevorzugt von 2:1 bis 10:7.

Überraschenderweise wurde festgestellt, dass die Waschleistung der erfindungsgemäßen Mischungen derjenigen der Rhamnolipide aber auch derjenigen der kationischen Tenside der allgemeinen Formel (I) überlegen ist. Die Mischungen eignen sich zur Herstellung von Wasch-, Reinigungs- und Haarbehandlungsmitteln, beispielsweise für die Herstellung lösungsmittelfreier flüssiger Mittel mit Aktivstoffgehalten bis zu 100 Gew.-%. Im Unterschied zu manchen anderen Aniontensid-Kationtensid-Kombinationen bilden die Mischungen im Wesentlichen keine schwer- oder unlöslichen Niederschläge.

Rhamnolipide sind Verbindungen, in denen eine Mono- oder Dirhamnoseeinheit glycosidisch mit der Hydroxylgruppe einer β-hydroxylgruppenhaltigen Fettsäure verbunden ist, wobei die Fettsäure mit einer Hydroxylgruppe eines weiteren hydroxylgruppenhaltigen Fettsäuremoleküls verestert sein kann. Man erhält sie durch Fermentation von Bakterien der Gattung Pseudomonas, insbesondere von Pseudomonas aeruginosa, bevorzugt bei deren Wachstum auf hydrophoben Substraten wie n-Alkanen oder Pflanzenölen. Rhamnolipide gehören wegen ihres oberflächenaktiven Verhaltens und ihrer Herkunft zu den sogenannten Biotensiden. Beispiele für Rhamnolipide sind:

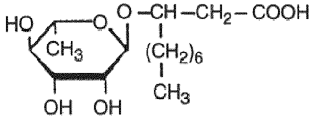

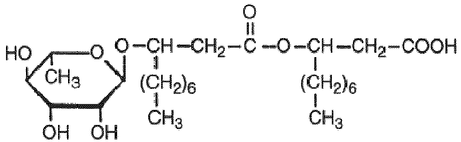

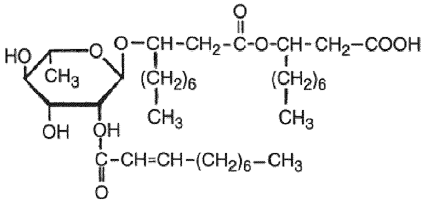

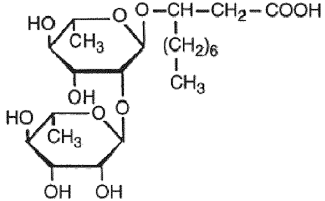

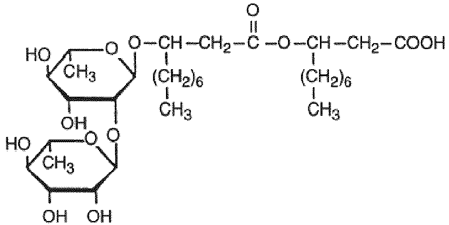

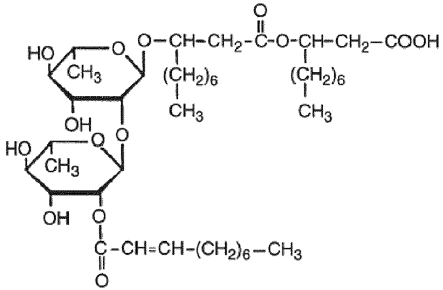

Man unterscheidet Mono-Rhamnolipide, in denen eine Rhamnoseeinheit vorhanden ist, von Di-Rhamnolipiden, die zwei Rhamnoseeinheiten besitzen. In üblichen Rhamnolipiden können neben C₁₀-Carbonsäuren und/oder -Hydroxycarbonsäuren auch längerkettige, beispielsweise C₁₂-Carbonsäuren und/oder -Hydroxycarbonsäuren, vorliegen.

Bevorzugt ist, wenn in den erfindungsgemäßen Mischungen der Rhamnolipid-Anteil zu mindestens 50 Gew.-%, insbesondere zu mindestens 90 Gew.-% und gegebenenfalls zu 100 Gew.-% aus Di-Rhamnolipid besteht.

In einer Ausführungsform der Erfindung besitzen in den Kationen N⁺(R¹R²R³R⁴) der Verbindungen der allgemeinen Formel (I) vorzugsweise zwei bis drei, insbesondere drei der vier Reste eine C-Kettenlänge von höchstens 4, insbesondere von 1 bis 2. Der übrige Rest beziehungsweise die anderen beiden Reste besitzen eine längere Kohlenstoffkette.

Die erfindungsgemäßen Mischungen weisen eine relativ niedrige Oberflächenspannung, die normalerweise im Bereich von 30 mN/m bis 40 mN/m liegt, und eine relativ niedrige kritische Micellkonzentration, die normalerweise im Bereich von 0,001 mmol/l bis 0,02 mmol/l liegt, auf. Sie sind gut wasserlöslich.

Wie schon ausgeführt eignen sich erfindungsgemäßen Mischungen aufgrund ihrer physikalischen Eigenschaften in besonderer Weise als Bestandteile von Wasch-, Reinigungs- oder Haarbehandlungsmitteln, insbesondere von flüssigen Wasch-, Reinigungs- oder Haarbehandlungsmitteln. Wegen ihrer hohen Wasch- beziehungsweise Reinigungskraft kann in solchen Mitteln die Tensidmenge bei gleichbleibender Leistung abgesenkt werden, oder man benötigt eine geringere Menge an den Mitteln, um ein gutes Wasch- oder Reinigungsergebnis zu erzielen. Ein Wasch-, Reinigungs- oder Haarbehandlungsmittel, enthaltend eine oben definierte Mischung, ist daher ein weiterer Gegenstand der Erfindung.

Als Waschmittel wird eine Zusammensetzung bezeichnet, welche zur Reinigung oder Pflege von Textilien geeignet ist und mindestens einen textilreinigenden oder textilpflegenden Aktivstoff enthält. Beispielhafte textilreinigende Aktivstoffe sind Tenside, insbesondere nichtionische und anionische Tenside, und Gerüststoffe, wie Phosphonate oder wasserenthärtende Polymere. Beispielhafte textilpflegende Aktivstoffe sind Duftstoffe, insbesondere verkapselte Duftstoffe, Textilweichmacher, insbesondere kationische Tenside und kationische Polymere, und Farbstoffe, insbesondere Textilfärbemittel.

Ein weiterer Erfindungsgegenstand ist ein Verfahren zum Waschen von Textilien, in dessen Verlauf eine oben definierte erfindungsgemäße Mischung oder ein Waschmittel, enthaltend eine oben definierte erfindungsgemäße Mischung, in eine Waschflotte eingebracht wird, die ein reinigungsbedürftiges Textil enthält oder in die ein reinigungsbedürftiges Textil eingebracht wird.

Als Reinigungsmittel wird eine Zusammensetzung bezeichnet, welche zur Reinigung und Pflege harter Oberflächen geeignet ist und mindestens einen oberflächenreinigenden oder oberflächenpflegenden Aktivstoff enthält. Beispielhafte oberflächenreinigende Aktivstoffe sind Tenside, insbesondere nichtionische und anionische Tenside, und Gerüststoffe, wie Phosphonate oder wasserenthärtende Polymere. Beispielhafte oberflächenpflegende Aktivstoffe sind Korrosionsinhibitoren, wie beispielsweise Zinksalze, und Hydrophobierungsmittel, insbesondere polymere Hydrophobierungsmittel.

Ein Verfahren zur Reinigung von harten Oberflächen, in dessen Verlauf eine oben definierte erfindungsgemäße Mischung oder ein Reinigungsmittel, enthaltend eine oben definierte erfindungsgemäße Mischung, in eine Reinigungsflotte eingebracht wird, die mit der zu reinigenden harten Oberfläche in Kontakt gebracht wird, ist ein weiterer Gegenstand der Erfindung.

Als Haarbehandlungsmittel wird eine Zusammensetzung bezeichnet, welche zur Reinigung und Pflege von Haaren, insbesondere von menschlichen Haaren, geeignet ist. Unter Haarbehandlungsmitteln sind insbesondere Haarreinigungsmittel wie Shampoos, Haarpflegemittel wie Haarkuren, Spülungen oder Haarpflegesprays sowie Haarstylingmittel wie Haargele, Haarsprays oder Haarwachse zu verstehen. Bevorzugt handelt es sich bei dem Haarbehandlungsmittel um ein Shampoo oder ein Haarpflegemittel.

Ein Verfahren zur Haarbehandlung, in dessen Verlauf eine oben definierte erfindungsgemäße Mischung oder ein Haarbehandlungsmittel, enthaltend eine oben definierte erfindungsgemäße Mischung, auf insbesondere nasse Haare aufgebracht wird, ist ein weiterer Gegenstand der Erfindung. Soweit es sich bei dem Haarbehandlungsmittel um ein Shampoo, eine Haarspülung oder eine Haarkur handelt, wird es vorzugsweise für mindestens 5 Sekunden, vorzugsweise für einen Zeitraum von 5 Sekunden bis 5 Minuten, auf das Haar einwirken gelassen und anschließend mit Wasser ausgespült.

Ein erfindungsgemäßes Wasch-, Reinigungs- oder Haarbehandlungsmittel enthält vorzugsweise 0,1 Gew.-% bis 40 Gew.-%, insbesondere 0,2 Gew.-% bis 30 Gew.-% und besonders bevorzugt 0,5 Gew.-% bis 15 Gew.-% der oben definierten erfindungsgemäßen Mischung. Es kann neben der genannten Mischung noch weitere herkömmliche Bestandteile solcher Mittel enthalten.

Im Rahmen der vorliegenden Erfindung enthält ein Wasch- oder Reinigungsmittel vorzugsweise zusätzlich zu der oben definierten erfindungsgemäßen Mischung einen oder mehrere Stoffe aus der Gruppe der nichtionische Tenside, anionischen Tenside, Gerüststoffe (Builder), Bleichmittel, Bleichaktivatoren, Enzyme, Elektrolyte, pH-Stellmittel, Parfüme, Parfümträger, Fluoreszenzmittel, Farbstoffe, Hydrotope, Schauminhibitoren, Antiredepositionsmittel, Vergrauungsinhibitoren, Einlaufverhinderer, Knitterschutzmittel, Farbübertragungsinhibitoren, antimikrobiellen Wirkstoffe, nicht-wässrigen Lösungsmittel, Germizide, Fungizide, Antioxidantien, Konservierungsmittel, Korrosionsinhibitoren, Antistatika, Bittermittel, Bügelhilfsmittel, Phobier- und Imprägniermittel, Hautpflegende Wirkstoffe, Quell- und Schiebefestmittel, weichmachenden Komponenten sowie UV-Absorber.

Im Rahmen der vorliegenden Erfindung enthält ein Haarbehandlungsmittel vorzugsweise zusätzlich zu der oben definierten erfindungsgemäßen Mischung einen oder mehrere Stoffe aus der Gruppe der nichtionischen Tenside, amphoteren/zwitterionischen Tenside, nichtionischen Polymere, anionischen Polymere, Aminosäuren, Oligopetide, Vitamine, Provitamine, Vitaminvorstufen, Betaine, Biochinone, Purin(derivate), Taurin(derivate), L-Carnitin(salze), Panthenol, Panthothensäure, 2-Furanone, 2-Furanonderivate, Ectoin, Allantoin, Pflanzenextrakte, Esteröle, UV-Lichtschutzfilter, Strukturierungsmittel, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle, und/oder Konservierungsmittel.

Ein erfindungsgemäßes Wasch-, Reinigungs- oder Haarbehandlungsmittel kann vorzugsweise bis zu 30 Gew.-% weiteres Tensid enthalten, wobei die zusätzlich vorhandenen Tenside vorzugsweise ebenfalls aus nachwachsenden Rohstoffen erhältlich sind. In einer besonderen Ausführungsform der Erfindung enthält das Mittel bis zu 20 Gew.-%, insbesondere 0,2 Gew-% bis 15 Gew.-% nichtionisches Tensid.

Geeignete nichtionische Tenside umfassen alkoxylierte Fettsäurealkylester, Fettsäureamide, alkoxylierte Fettsäureamide, Polyhydroxyfettsäureamide, Alkylphenolpolyglycolether, Aminoxide, Alkylpolyglycoside und Mischungen daraus. Außerdem können als weitere nichtionische Tenside auch Alkylglykoside der allgemeinen Formel R⁵O(G)ₓ eingesetzt werden, in der R⁵ einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse nichtionischer Tenside, die entweder als alleiniges nichtionisches Tensid oder in Kombination mit anderen zusätzlichen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethyl-aminoxid und N-Talgalkyl-N,N-dihydroxyethylaminoxid, und der Fettsäurealkanolamide können eingesetzt werden.

Weitere gewünschtenfalls geeignete Tenside sind Polyhydroxyfettsäureamide der Formel, 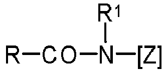 in der R für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgender Acylierung mit einer Fettsäure, einem Fettsäurealkylester oder einem Fettsäurechlorid erhalten werden können. Zur Gruppe der Polyhydroxyfettsäureamide gehören auch Verbindungen der Formel 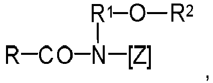 in der R für einen linearen oder verzweigten Alkyl- oder Alkenylrest mit 7 bis 12 Kohlenstoffatomen, R¹ für einen linearen, verzweigten oder zyklischen Alkylrest oder einen Arylrest mit 2 bis 8 Kohlenstoffatomen und R² für einen linearen, verzweigten oder zyklischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können durch Umsetzung mit Fettsäuremethylestern in Gegenwart eines Alkoxids als Katalysator in die gewünschten Polyhydroxyfettsäureamide überführt werden.

Ein erfindungsgemäßes Wasch- oder Reinigungsmittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Glycindiessigsäure, Methylglycindiessigsäure, Nitrilotriessigsäure, Iminodisuccinate wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendia-mintetrakis(methylenphosphonsäure), Lysintetra(methylenphosphonsäure) und 1-Hydroxyethan-1,1-diphosphonsäure, polymere Hydroxyverbindungen wie Dextrin sowie polymere (Poly-)carbonsäuren, insbesondere durch Oxidation von Polysacchariden zugängliche Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative mittlere Molekülmasse der Homopolymeren ungesättigter Carbonsäuren liegt im Allgemeinen zwischen 5 000 g/mol und 200 000 g/mol, die der Copolymeren zwischen 2 000 g/mol und 200 000 g/mol, vorzugsweise 50 000 g/mol bis 120 000 g/mol, jeweils bezogen auf freie Säure. Ein besonders bevorzugtes Acrylsäure-Maleinsäure-Copolymer weist eine relative mittlere Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäue, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure oder Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative mittlere Molekülmasse zwischen 1 000 g/mol und 200 000 g/mol, vorzugsweise zwischen 200 g/mol und 50 000 g/mol auf. Weitere bevorzugte Copolymere sind solche, die als Monomere Acrolein und Acrylsäure/ Acrylsäuresalze beziehungsweise Vinylacetat aufweisen. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen in der oberen Hälfte der genannten Bereiche werden vorzugsweise in pastenförmigen oder flüssigen, insbesondere wasserhaltigen, Wasch- oder Reinigungsmitteln eingesetzt.

Als wasserlösliche anorganische Buildermaterialien kommen insbesondere Polyphosphate, vorzugsweise Natriumtriphosphat, in Betracht. Als wasserunlösliche anorganische Buildermaterialien werden insbesondere kristalline oder amorphe, wasserdispergierbare Alkalialumosilikate, in Mengen nicht über 25 Gew.-%, vorzugsweise von 3 Gew.-% bis 20 Gew.-% und insbesondere in Mengen von 5 Gew.-% bis 15 Gew.-% eingesetzt. Unter diesen sind die kristallinen Natriumalumosilikate in Waschmittelqualität, insbesondere Zeolith A, Zeolith P sowie Zeolith MAP und gegebenenfalls Zeolith X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen liegt in der Regel im Bereich von 100 bis 200 mg CaO pro Gramm.

Zusätzlich oder alternativ zum genannten wasserunlöslichen Alumosilikat und Alkalicarbonat können weitere wasserlösliche anorganische Buildermaterialien enthalten sein. Zu diesen gehören neben den Polyphosphaten wie Natriumtriphosphat insbesondere die wasserlöslichen kristallinen und/oder amorphen Alkalisilikat-Builder. Derartige wasserlösliche anorganische Buildermaterialien sind in den Mitteln vorzugsweise in Mengen von 1 Gew.-% bis 20 Gew.-%, insbesondere von 5 Gew.-% bis 15 Gew.-% enthalten. Die als Buildermaterialien brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in den Mitteln eingesetzt werden. In einer weiteren bevorzugten Ausführungsform wird ein kristallines Natriumschichtsilikat mit einem Modul von 2 bis 3 eingesetzt, wie es aus Sand und Soda hergestellt werden kann. Natriumsilikate mit einem Modul im Bereich von 1,9 bis 3,5 werden in einer weiteren Ausführungsform eingesetzt. In einer bevorzugten Ausgestaltung solcher Mittel setzt man ein granulares Compound aus Alkalisilikat und Alkalicarbonat ein, wie es zum Beispiel unter dem Namen Nabion^{®} 15 im Handel erhältlich ist.

Als in Wasch- und Reinigungsmitteln geeignete peroxidische Bleichmittel kommen insbesondere organische Persäuren oder persaure Salze organischer Säuren, wie Phthalimidopercapronsäure, Perbenzoesäure, Monoperoxyphthalsäure, und Diperdodecandisäure sowie deren Salze wie Magnesiummonoperoxyphthalat, Diacylperoxide, Wasserstoffperoxid und unter den Einsatzbedingungen Wasserstoffperoxid abgebende anorganische Salze, wie Alkaliperborat, Alkalipercarbonat und/oder Alkalipersilikat, und Wasserstoffperoxid-Einschlussverbindungen, wie H₂O₂-Harnstoffaddukte, sowie Mischungen aus diesen in Betracht. Wasserstoffperoxid kann dabei auch mit Hilfe eines enzymatischen Systems, das heißt einer Oxidase und ihres Substrats, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder Wasserstoffperoxid eingesetzt. Ein im Rahmen der Erfindung einsetzbares Waschmittel enthält peroxidisches Bleichmittel in Mengen von vorzugsweise bis zu 60 Gew.-%, insbesondere von 5 Gew.-% bis 50 Gew.-% und besonders bevorzugt von 15 Gew.-% bis 30 Gew.-% oder alternativ von 2,5 Gew.-% bis 20 Gew.-%, wobei in flüssigen Mitteln Wasserstoffperoxid und in festen Mitteln Natriumpercarbonat das besonders bevorzugte peroxidische Bleichmittel ist. Vorzugsweise weisen peroxidische Bleichmittel-Partikel eine Teilchengröße im Bereich von 10 µm bis 5000 µm, insbesondere von 50 µm bis 1000 µm und/oder eine Dichte von 0,85 g/cm³ bis 4,9 g/cm³, insbesondere von 0,91 g/cm³ bis 2,7 g/cm³ auf.

Als bleichaktivierende, unter Perhydrolysebedingungen Peroxocarbonsäure-liefernde Verbindung können insbesondere Verbindungen, die unter Perhydrolysebedingungen gegebenenfalls substituierte Perbenzoesäure und/oder aliphatische Peroxocarbonsäuren mit 1 bis 12 C-Atomen, insbesondere 2 bis 4 C-Atomen ergeben, allein oder in Mischungen, eingesetzt werden. Geeignet sind Bleichaktivatoren, die O- und/oder N-Acylgruppen insbesondere der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Glykolurile, insbesondere Tetraacetylglykoluril (TAGU), acylierte Triazinderivate, insbesondere 1,5-Diacetyl-2,4-dioxohexahydro-1,3,5-triazin (DADHT), N-Acylimide, insbesondere N-Nonanoylsuccinimid (NOSI), acylierte Phenolsulfonate oder -carboxylate beziehungsweise die Sulfon- oder Carbonsäuren von diesen, insbesondere Nonanoyl- oder Isononanoyl- oder Lauroyloxybenzolsulfonat (NOBS beziehungsweise iso-NOBS beziehungsweise LOBS) oder Decanoyloxybenzoat (DOBA), deren formale Kohlensäureesterderivate wie 4-(2-Decanoyloxyethoxycarbonyloxy)-benzolsulfonat (DECOBS), acylierte mehrwertige Alkohole, insbesondere Triacetin, Ethylenglykoldiacetat und 2,5-Di-acetoxy-2,5-dihydrofuran sowie acetyliertes Sorbitol und Mannitol und deren Mischungen (SORMAN), acylierte Zuckerderivate, insbesondere Pentaacetylglukose (PAG), Pentaacetyl-fruktose, Tetraacetylxylose und Octaacetyllactose, acetyliertes, gegebenenfalls N-alkyliertes Glucamin und Gluconolacton, und/oder N-acylierte Lactame, beispielsweise N-Benzoylcaprolactam.

Zusätzlich zu den Verbindungen, die unter Perhydrolysebedingungen Peroxocarbonsäuren bilden, oder an deren Stelle können weitere bleichaktivierende Verbindungen, wie beispielsweise Nitrile, aus denen sich unter Perhydrolysebedingungen Perimidsäuren bilden, vorhanden sein. Dazu gehören insbesondere Aminoacetonitrilderivate mit quaterniertem Stickstoffatom gemäß der Formel 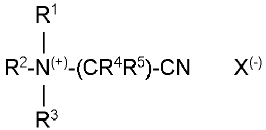 in der R¹ für -H, -CH₃, einen C₂₋₂₄-Alkyl- oder -Alkenylrest, einen substituierten C₁₋₂₄-Alkyl- oder C₂₋₂₄-Alkenylrest mit mindestens einem Substituenten aus der Gruppe -Cl, -Br, -OH, -NH₂, -CN und - N⁽⁺⁾-CH₂-CN, einen Alkyl- oder Alkenylarylrest mit einer C₁₋₂₄-Alkylgruppe, oder für einen substituierten Alkyl- oder Alkenylarylrest mit mindestens einer, vorzugsweise zwei, gegebenenfalls substituierten C₁₋₂₄-Alkylgruppe(n) und gegebenenfalls weiteren Substituenten am aromatischen Ring steht, R² und R³ unabhängig voneinander ausgewählt sind aus -CH₂-CN, -CH₃, -CH₂-CH₃, CH₂-CH2-CH₃, - CH(CH₃)-CH₃,-CH₂-OH, -CH₂-CH₂-OH, -CH(OH)-CH₃, -CH₂-CH₂-CH₂-OH, -CH₂-CH(OH)-CH₃, - CH(OH)-CH₂-CH₃, -(CH₂CH₂-O)ₙH mit n = 1, 2, 3, 4, 5 oder 6, R⁴ und R⁵ unabhängig voneinander eine voranstehend für R¹, R² oder R³ angegebene Bedeutung haben, wobei mindestens 2 der genannten Reste, insbesondere R² und R³, auch unter Einschluss des Stickstoffatoms und gegebenenfalls weiterer Heteroatome ringschließend miteinander verknüpft sein können und dann vorzugsweise einen Morpholino-Ring ausbilden, und X ein ladungsausgleichendes Anion, vorzugsweise ausgewählt aus Benzolsulfonat, Toluolsulfonat, Cumolsulfonat, den C₉₋₁₅-Alkylbenzolsulfonaten, den C₁₋₂₀-Alkylsulfaten, den C₈₋₂₂-Carbonsäure-methylestersulfonaten, Sulfat, Hydrogensulfat und deren Gemischen, ist, können eingesetzt werden. Unter Perhydrolysebedingungen Peroxocarbonsäuren oder Perimidsäuren bildende Bleichaktivatoren sind vorzugsweise in Mengen bis zu 25 Gew.-%, insbesondere 0,1 Gew.-% bis 10 Gew.-% in erfindungsgemäßen Mitteln vorhanden. Vorzugsweise weisen Bleichaktivator-Partikel eine Teilchengröße im Bereich von 10 µm bis 5000 µm, insbesondere von 50 µm bis 1000 µm und/oder eine Dichte von 0,85 g/cm³ bis 4,9 g/cm³, insbesondere von 0,91 g/cm³ bis 2,7 g/cm³ auf.

Die Anwesenheit von bleichkatalysierenden Übergangsmetallkomplexen, zusätzlich zu oder an Stelle von den genannten Bleichaktivatoren, ist möglich. Diese werden vorzugsweise unter den Cobalt-, Eisen-, Kupfer-, Titan-, Vanadium-, Mangan- und Rutheniumkomplexen ausgewählt. Als Liganden in derartigen Übergangsmetallkomplexen kommen sowohl anorganische als auch organische Verbindungen in Frage, zu denen neben Carboxylaten insbesondere Verbindungen mit primären, sekundären und/oder tertiären Amin- und/oder Alkohol-Funktionen, wie Pyridin, Pyridazin, Pyrimidin, Pyrazin, Imidazol, Pyrazol, Triazol, 2,2'-Bispyridylamin, Tris-(2-pyridylmethyl)amin, 1,4,7-Triazacyclononan, 1,4,7-Trimethyl-1,4,7-triazacyclononan, 1,5,9-Trimethyl-1,5,9-triazacyclododecan, (Bis-((1-methylimidazol-2-yl)-methyl))-(2-pyridylmethyl)-amin, N,N'-(Bis-(1-methylimidazol-2-yl)-methyl)-ethylendiamin, N-Bis-(2-benzimidazolylmethyl)-aminoethanol, 2,6-Bis-(bis-(2-benzimidazolylmethyl)aminomethyl)-4-methylphenol, N,N,N',N'-Tetrakis-(2-benzimidazolylmethyl)-2-hydroxy-1,3-diaminopropan, 2,6-Bis-(bis-(2-pyridylmethyl)aminomethyl)-4-methylphenol, 1,3-Bis-(bis-(2-benzimidazolyl-methyl)aminomethyl)-benzol, Sorbitol, Mannitol, Erythritol, Adonitol, Inositol, Lactose, und gegebenenfalls substituierte Salene, Porphine und Porphyrine gehören. Zu den anorganischen Neutralliganden gehören insbesondere Ammoniak und Wasser. Falls nicht sämtliche Koordinationsstellen des Übergangsmetallzentralatoms durch Neutralliganden besetzt sind, enthält der Komplex weitere, vorzugsweise anionische und unter diesen insbesondere ein- oder zweizähnige Liganden. Zu diesen gehören insbesondere die Halogenide wie Fluorid, Chlorid, Bromid und lodid, und die (NO₂)⁻-Gruppe, das heißt ein Nitro-Ligand oder ein Nitrito-Ligand. Die (NO₂)⁻-Gruppe kann an ein Übergangsmetall auch chelatbildend gebunden sein oder sie kann zwei Übergangsmetallatome asymmetrisch oderµ1-O-verbrücken. Außerden genannten Liganden können die Übergangsmetallkomplexe noch weitere, in der Regel einfacher aufgebaute Liganden, insbesondere ein- oder mehrwertige Anionliganden, tragen. In Frage kommen beispielsweise Nitrat, Acetat, Trifluoracetat, Formiat, Carbonat, Citrat, Oxalat, Perchlorat sowie komplexe Anionen wie Hexafluorophosphat. Die Anionliganden sollen für den Ladungsausgleich zwischen Übergangsmetall-Zentralatom und dem Ligandensystem sorgen. Auch die Anwesenheit von Oxo-Liganden, Peroxo-Liganden und Imino-Liganden ist möglich. Insbesondere derartige Liganden können auch verbrückend wirken, so dass mehrkernige Komplexe entstehen. Im Falle verbrückter, zweikerniger Komplexe müssen nicht beide Metallatome im Komplex gleich sein. Auch der Einsatz zweikerniger Komplexe, in denen die beiden Übergangsmetallzentralatome unterschiedliche Oxidationszahlen aufweisen, ist möglich. Falls Anionliganden fehlen oder die Anwesenheit von Anionliganden nicht zum Ladungsausgleich im Komplex führt, sind in den gemäß der Erfindung zu verwendenden Übergangsmetallkomplex-Verbindungen anionische Gegenionen anwesend, die den kationischen Übergangsmetall-Komplex neutralisieren. Zu diesen anionischen Gegenionen gehören insbesondere Nitrat, Hydroxid, Hexafluorophosphat, Sulfat, Chlorat, Perchlorat, die Halogenide wie Chlorid oder die Anionen von Carbonsäuren wie Formiat, Acetat, Oxalat, Benzoat oder Citrat. Beispiele für einsetzbare Übergangsmetallkomplex-Verbindungen sind [N,N'-Bis[(2-hydroxy-5-vinylphenyl)-methylen]-1,2-diamino-cyclohexan]-mangan-(III)-chlorid, [N,N'-Bis[(2-hydroxy-5-nitrophenyl)-methylen]-1,2-diamino-cyclohexan]-mangan-(III)-acetat, [N,N'-Bis[(2-hydroxyphenyl)-methylen]-1,2-phenylendiamin]-mangan-(III)-acetat, [N,N'-Bis[(2-hydroxyphenyl)-methylen]-1,2-diaminocyclohexan]-mangan-(III)-chlorid, [N,N'-Bis[(2-hydroxyphenyl)-methylen]-1,2-diaminoethan]-mangan-(III)-chlorid, [N,N'-Bis[(2-hydroxy-5-sulfonatophenyl)-methylen]-1,2-diaminoethan]-mangan-(III)-chlorid, Mangan-oxalatokomplexe, Nitropentammin-cobalt(III)-chlorid, Nitritopentammin-cobalt(III)-chlorid, Hex-ammincobalt(III)-chlorid, Chloropentammin-cobalt(III)-chlorid sowie der Peroxo-Komplex [(NH₃)₅Co-O-O-Co(NH₃)₅]Cl₄.

Als in Wasch- und Reinigungsmitteln verwendbare Enzyme kommen solche aus der Klasse der Proteasen, Amylasen, Lipasen, Cutinasen, Pullulanasen, Hemicellulasen, Cellulasen, Oxidasen, Laccasen und Peroxidasen sowie deren Gemische in Frage. Besonders geeignet sind aus Pilzen oder Bakterien, wie Bacillus subtilis, Bacillus licheniformis, Bacillus Lenaus, Streptomyces greises, Humicola lanuginosa, Humicola insolens, Pseudomonas pseudoalcaligenes, Pseudomonas cepacia oder Coprinus cinereus gewonnene enzymatische Wirkstoffe. Die Enzyme können an Trägerstoffen adsorbiert und/oder in Hüllsubstanzen eingebettet sein, um sie gegen vorzeitige Inaktivierung zu schützen. Sie sind in den erfindungsgemäßen Wasch- oder Reinigungsmitteln vorzugsweise in Mengen bis zu 5 Gew.-%, insbesondere von 0,002 Gew.-% bis 4 Gew.-%, enthalten. Falls das erfindungsgemäße Mittel Protease enthält, weist es vorzugsweise eine proteolytische Aktivität im Bereich von etwa 100 PE/g bis etwa 10 000 PE/g, insbesondere 300 PE/g bis 8000 PE/g auf. Falls mehrere Enzyme in dem erfindungsgemäßen Mittel eingesetzt werden sollen, kann dies durch Einarbeitung der zwei oder mehreren separaten beziehungsweise in bekannter Weise separat konfektionierten Enzyme oder durch zwei oder mehrere gemeinsam in einem Granulat konfektionierte Enzyme durchgeführt werden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Aminosäuren wie beispielsweise Glycin, Glutaminsäure, Arginin und/oder Asparaginsäure, Glykolsäure, Maleinsäure, Fumarsäure, Salicylsäure, Bernsteinsäure, Glutarsäure und/oder Adipinsäure, aber gegebenenfalls auch Mineralsäuren, insbesondere Schwefelsäure, oder Basen, insbesondere Ammonium- oder Alkalihydroxide, enthalten. Derartige pH-Regulatoren sind in erfindungsgemäßen Wasch- und Reinigungsmitteln in Mengen von vorzugsweise nicht über 20 Gew.-%, insbesondere von 1,2 Gew.-% bis 17 Gew.-%, enthalten. In Haarbehandlungsmitteln werden vorzugsweise organische Säuren eingesetzt. Besonders bevorzugt werden Milchsäure und/oder Maleinsäure als organische Säure eingesetzt. Die organischen Säuren können dazu dienen, den pH-Wert des Haarbehandlungsmittels insbesondere auf einen Wert zwischen 3,5 und 5 einzustellen. In einer bevorzugten Ausführungsform weist das Haarbehandlungsmittel einen pH-Wert im Bereich von 3,75 bis 4,75 auf.

Vergrauungsinhibitoren haben die Aufgabe, den von Textilfasern abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf das Waschmittel, eingesetzt.

Waschmittel können gewünschtenfalls einen üblichen Farbübertragungsinhibitor, diesen dann vorzugsweise in Mengen bis zu 2 Gew.-%, insbesondere 0,1 Gew.-% bis 1 Gew.-%, enthalten, der in einer bevorzugten Ausgestaltung ausgewählt wird aus den Polymeren aus Vinylpyrrolidon, Vinylimidazol, Vinylpyridin-N-Oxid oder den Copolymeren aus diesen. Brauchbar sind sowohl Polyvinylpyrrolidone mit Molgewichten von 15 000 g/mol bis 50 000 g/mol wie auch Polyvinylpyrrolidone mit höheren Molgewichten von beispielsweise bis zu über 1 000 000 g/mol, insbesondere von 1 500 000 g/mol bis 4 000 000 g/mol, N-Vinylimidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polyamin-N-Oxid-Polymere und Polyvinylalkohole. Eingesetzt werden können aber auch enzymatische Systeme, umfassend eine Peroxidase und Wasserstoffperoxid beziehungsweise eine in Wasser Wasserstoffperoxid-liefernde Substanz. Der Zusatz einer Mediatorverbindung für die Peroxidase, zum Beispiel eines Acetosyringons, eines Phenolderivats oder eines Phenotiazins oder Phenoxazins, ist in diesem Fall bevorzugt, wobei auch zusätzlich obengenannte polymere Farbübertragungsinhibitorwirkstoffe eingesetzt werden können. Polyvinylpyrrolidon weist vorzugsweise eine durchschnittliche Molmasse im Bereich von 10 000 g/mol bis 60 000 g/mol, insbesondere im Bereich von 25 000 g/mol bis 50 000 g/mol auf. Unter den Copolymeren sind solche aus Vinylpyrrolidon und Vinylimidazol im Molverhältnis 5:1 bis 1:1 mit einer durchschnittlichen Molmasse im Bereich von 5000 g/mol bis 50 000 g/mol, insbesondere 10 000 g/mol bis 20 000 g/mol bevorzugt. In bevorzugten Ausführungsformen der Erfindung sind die Waschmittel allerdings frei von derartigen zusätzlichen Farbübertragungsinhibitoren.

Waschmittel können als optische Aufheller beispielsweise Derivate der Diaminostilbendisulfonsäure beziehungsweise deren Alkalimetallsalze enthalten, obgleich sie für den Einsatz als Colorwaschmittel vorzugsweise frei von optischen Aufhellern sind. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder4-(4-Chlorstyryl)-4'-(2-sulfostyryl)-diphenyls. Auch Gemische der vorgenannten optischen Aufheller können verwendet werden.

Insbesondere beim Einsatz in maschinellen Verfahren kann es von Vorteil sein, den Waschmitteln übliche Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

In einer bevorzugten Ausführungsform ist ein erfindungsgemäßes Wasch- oder Reinigungsmittel teilchenförmig und enthält neben dem erfindungswesentlichen Tensid Builder, insbesondere in einer Menge im Bereich von 1 Gew.-% bis 60 Gew.-%.

In einer weiteren bevorzugten Ausführungsform ist ein erfindungsgemäßes Wasch- oder Reinigungsmittel flüssig und enthält bis zu 90 Gew.-%, insbesondere 10 Gew.-% bis 85 Gew.-%, bevorzugt 25 Gew.-% bis 75 Gew.-%, und besonders bevorzugt 35 Gew.-% bis 65 Gew.-% Wasser, wassermischbares Lösungsmittel oder eines Gemisches aus Wasser und wassermischbarem Lösungsmittel. Zu wassermischbaren Lösungsmitteln gehören beispielsweise einwertige Alkohole mit 1 bis 4 C-Atomen, insbesondere Methanol, Ethanol, Isopropanol und tert.-Butanol, Diole und Triole mit 2 bis 4 C-Atomen, insbesondere Ethylenglykol, Propylenglykol und Glycerin, sowie deren Gemische und die aus den genannten Verbindungsklassen ableitbaren Ether. Derartige wassermischbare Lösungsmittel sind in den erfindungsgemäßen Wasch- oder Reinigungsmitteln vorzugsweise in Mengen nicht über 30 Gew.-%, insbesondere von 2 Gew.-% bis 20 Gew.-%, vorhanden. Ein flüssiges Wasch- oder Reinigungsmittel weist vorzugsweise einen pH-Wert von mindestens pH 5,6, insbesondere im Bereich von pH 7 bis pH 9, auf.

Ein erfindungsgemäßes Haarbehandlungsmittel ist vorzugsweise flüssig und enthält einen wässrigen oder einen wässrig-alkoholischen Träger. Ein wässriger Träger enthält mindestens 50 Gew.-% Wasser. Unter wässrig-alkoholischen Trägern sind wässrige Lösungen enthaltend 3 bis 70 Gew.-% eines C₂-C₆-Alkohols, insbesondere Ethanol oder Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

In einer weiteren bevorzugten Ausführungsform liegt ein erfindungsgemäßes Mittel einzeldosierfertig portioniert in einer aus wasserlöslichem Material gebildeten Kammer vor; dann enthält das Mittel vorzugsweise weniger als 15 Gew.-%, insbesondere im Bereich von 1 Gew.-% bis 12 Gew.-% Wasser. Eine Portion ist eine eigenständige Dosiereinheit mit mindestens einer Kammer, in der zu dosierendes Gut enthalten ist. Eine Kammer ist ein durch Wandungen (zum Beispiel durch eine Folie) abgegrenzter Raum, welcher auch ohne das zu dosierende Gut (gegebenenfalls unter Veränderung seiner Form) existieren kann. Bei einer Oberflächenbeschichtung oder einer Schicht einer Oberflächenbeschichtung handelt es sich somit nicht um eine Wandung gemäß der vorliegenden Erfindung.

Dabei sind die Wandungen der Kammer aus einem wasserlöslichen Material. Die Wasserlöslichkeit des Materials kann mit Hilfe eines in einem quadratischen Rahmen (Kantenlänge auf der Innenseite: 20 mm) fixierten quadratischen Films des besagten Materials (Film: 22 × 22 mm mit einer Dicke von 76 µm) nach dem folgenden Messprotokoll bestimmt werden. Besagter gerahmter Film wird in 800 ml auf 20 °C temperiertes, destilliertes Wasser in einem 1 Liter Becherglas mit kreisförmiger Bodenfläche (Fa. Schott, Mainz, Becherglas 1000 ml, niedrige Form) eingetaucht, so dass die Fläche des eingespannten Films im rechten Winkel zur Bodenfläche des Becherglases angeordnet ist, die Oberkante des Rahmens 1 cm unter der Wasseroberfläche ist und die Unterkante des Rahmens parallel zur Bodenfläche des Becherglases derart ausgerichtet ist, dass die Unterkante des Rahmens entlang des Radius der Bodenfläche des Becherglases verläuft und die Mitte der Unterkante des Rahmens über der Mitte des Radius des Becherglasbodens angeordnet ist. Das Material löst sich unter Rühren (Rührgeschwindigkeit Magnetrührer 300 rpm, Rührstab: 5 cm lang) innerhalb von 600 Sekunden derart auf, dass mit dem bloßen Auge keine einzelnen festförmigen Partikel mehr sichtbar sind.

Die Wandungen der Kammern und damit die wasserlöslichen Umhüllungen der erfindungsgemäßen Waschmittel werden vorzugsweise durch ein wasserlösliches Folienmaterial gebildet. Solche wasserlöslichen Verpackungen können entweder durch Verfahren des vertikalen Formfüllversiegelns oder durch Warmformverfahren hergestellt werden.

Das Warmformverfahren schließt im Allgemeinen das Formen einer ersten Lage aus einem wasserlöslichen Folienmaterial zum Bilden von Ausbuchtungen zum Aufnehmen einer Zusammensetzung darin, Einfüllen der Zusammensetzung in die Ausbuchtungen, Bedecken der mit der Zusammensetzung gefüllten Ausbuchtungen mit einer zweiten Lage aus einem wasserlöslichen Folienmaterial und Versiegeln der ersten und zweiten Lagen miteinander zumindest um die Ausbuchtungen herum ein.

Das wasserlösliche Folienmaterial wird vorzugsweise ausgewählt aus Polymeren oder Polymergemischen. Die Umhüllung kann aus einer oder aus zwei oder mehr Lagen von wasserlöslichem Folienmaterial gebildet werden. Die wasserlöslichen Folienmaterialien der ersten Lage und der weiteren Lagen, falls vorhanden, können gleich oder unterschiedlich sein.

Es ist bevorzugt, dass die wasserlösliche Umhüllung Polyvinylalkohol oder ein Polyvinylalkoholcopolymer enthält; besonders bevorzugt besteht sie aus Polyvinylalkohol oder Polyvinylalkoholcopolymer.

Wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 gmol⁻¹, vorzugsweise von 20.000 bis 500.000 gmol⁻¹, besonders bevorzugt von 30.000 bis 100.000 gmol⁻¹ und insbesondere von 40.000 bis 80.000 gmol⁻¹ liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Einem zur Herstellung der wasserlöslichen Umhüllung geeigneten Folienmaterial können zusätzlich Polymere, ausgewählt aus der Gruppe umfassend Acrylsäure-haltige Polymere, Polyacrylamide, Oxazolin-Polymere, Polystyrolsulfonate, Polyurethane, Polyester, Polyether, Polymilchsäure, und/oder Mischungen der vorstehenden Polymere, zugesetzt sein. Auch die Copolymerisation von solchen Polymeren zugrundeliegenden Monomeren, einzeln oder in Mischungen aus zweien oder mehreren, mit Vinylacetat ist möglich.

Bevorzugte Polyvinylalkoholcopolymere umfassen neben Vinylalkohol eine ethylenisch ungesättigte Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus; unter den Estern sind C₁₋₄-Alkylester oder -Hydroxyalkylester bevorzugt. Ebenso bevorzugte Polyvinylalkoholcopolymere umfassen neben Vinylalkohol als weitere Monomere ethylenisch ungesättigte Dicarbonsäuren. Geeignete Dicarbonsäure sind beispielsweise Itaconsäure, Maleinsäure, Fumarsäure und Mischungen daraus, wobei Itaconsäure besonders bevorzugt ist.

Geeignete wasserlösliche Folien zum Einsatz in den Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Die Wasch- oder Reinigungsmittelportion, umfassend das Wasch- oder Reinigungsmittel und die wasserlösliche Umhüllung, kann eine oder mehr Kammern aufweisen. Die wasserlöslichen Umhüllungen mit einer Kammer können eine im Wesentlichen formstabile kugel-, rotationsellipsoid-, würfel-, quader- oder kissenförmige Ausgestaltung mit einer kreisförmigen, elliptischen, quadratischen oder rechteckigen Grundform aufweisen. Das Mittel kann in einer oder mehreren Kammern, falls vorhanden, der wasserlöslichen Umhüllung enthalten sein.

In einer bevorzugten Ausführungsform weist die wasserlösliche Umhüllung zwei Kammern auf. In dieser Ausführungsform können beide Kammern jeweils eine feste Teilzusammensetzung oder jeweils eine flüssige Teilzusammensetzung enthalten, oder die erste Kammer enthält eine flüssige und die zweite Kammer eine feste Teilzusammensetzung.

Die Anteile der Mittel, die in den unterschiedlichen Kammern einer wasserlöslichen Umhüllung mit zwei oder mehr Kammern enthalten sind, können dieselbe Zusammensetzung aufweisen. Vorzugsweise weisen die Mittel in einer wasserlöslichen Umhüllung mit mindestens zwei Kammern jedoch Teilzusammensetzungen auf, die sich mindestens in einem Inhaltsstoff und/oder in dem Gehalt mindestens eines Inhaltsstoffes unterscheiden. Vorzugsweise weist eine Teilzusammensetzung solcher erfindungsgemäßer Mittel Enzym und/oder Bleichaktivator auf und eine getrennt davon vorliegende weitere Teilzusammensetzung weist peroxidisches Bleichmittel auf, wobei dann die erstgenannte Teilzusammensetzung insbesondere kein peroxidisches Bleichmittel und die zweitgenannte Teilzusammensetzung insbesondere kein Enzym und keinen Bleichaktivator aufweist.

Durch die portionsweise Verpackung in eine wasserlösliche Umhüllung wird der Anwender in die Lage versetzt, für eine Anwendung eine oder gewünschtenfalls mehrere, vorzugsweise eine, der Portionen in die Wasch- oder Geschirrspülmaschine, insbesondere in die Einspülkammer einer Waschmaschine, oder in ein Behältnis zur Durchführung eines manuellen Wasch- oder Reinigungsverfahrens zu geben. Derartige Portionspackungen erfüllen den Wunsch des Verbrauchers nach vereinfachter Dosierung. Nach Zugabe von Wasser löst sich das Umhüllungsmaterial auf, so dass die Inhaltsstoffe freigesetzt werden und in der Flotte ihre Wirkung entfalten können. Vorzugsweise wiegt eine wasserlöslich umhüllte Portion 10 g bis 35 g, insbesondere 12 g bis 28 g und besonders bevorzugt 12 g bis 15 g, wobei auf den in der Gewichtsangabe enthaltenen Anteil der wasserlöslichen Umhüllung 0,3 g bis 2,5 g, insbesondere 0,7 g bis 1,2 g entfallen.

Die Herstellung fester erfindungsgemäßer Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe wie zum Beispiel Bleichmittel gegebenenfalls später separat zugesetzt werden. Zur Herstellung von Mitteln mit erhöhtem Schüttgewicht, insbesondere im Bereich von 650 g/l bis 950 g/l, ist ein einen Extrusionsschritt aufweisendes Verfahren bevorzugt.

Flüssige beziehungsweise pastöse erfindungsgemäße Mittel in Form von Wasser übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

### Beispiele

### Beispiel 1: Waschleistung

In einer Hochdurchsatz-Waschvorrichtung wurden bei 30°C und pH 8 die Waschleistung eines Rhamnolipids (Rewoferm^{®} RL100) in Kombination mit dem Kationtensid C_{12/14}-dimethyl-hydroxyethyl-ammoniumchlorid (Präpagen^{®} HY) untersucht. Dabei wurde zum Vergleich das Rhamnolipid allein mit untersucht. Es wurden verschiedene molare Verhältnisse des Rhamnolipis zum Kationtensid untersucht (in der nachfolgenden Tabelle angegeben). Die Gesamttensidkonzentration lag bei allen Versuchen bei 0,62 g/l. Es wurden 12 verschiedene standardisierte Anschmutzungen (3*Lippenstift, Pigment/Öl, Pigment/Talg, Olivenöl, 2*Talg, Mayonnaise, Frittierfett, Schmalz, Rinderfett) auf Polyester-Baumwoll-Mischgewebe eingesetzt. Nach dem Trocknen wurden die Remissionswerte der gewaschenen Textilien bestimmt und addiert. In den nachfolgenden Tabellen sind die Ergebnisse als Mittelwerte von 3-fach Bestimmungen der 12 verschiedenen Anschmutzungen angegeben:

### Summe der Remissionswerte

| | |
|---|---|
| Rhamnolipid | 457,4 |
| Präpagen^{®} HY | * |
| Rhamnolipid / Präpagen^{®} HY 1:0,7 | 630,6 |
| Rhamnolipid / Präpagen^{®} HY 1:0,5 | 644,9 |
| Rhamnolipid / Präpagen^{®} HY 1:0,3 | 649,5 |
| Rhamnolipid / Präpagen^{®} HY 1:0,1 | 646,5 |

| | |
|---|---|
| * nicht im Detail als Zahlenwert bestimmt, lag unter dem Wert für das Rhamnolipid | |

Die Waschleistung der Mischungen lag deutlich über derjenigen der Einzelkomponenten.

## Patentansprüche

1. Mischung aus einem Rhamnolipid und einer Verbindung der allgemeinen Formel (I)
X^{a-} a N⁺(R¹R²R³R⁴) (I)
in der
X^{a-} ein Anion ist,
a für 1, 2 oder 3 steht und
R¹ ausgewählt ist aus R² und H, R² ausgewählt wird aus C₁- bis C₄-Alkylresten und C₂- bis C₄-Hydroxyalkylresten, R³ ausgewählt ist aus R⁴ und R², und R⁴ ausgewählt ist aus C₁₀- bis C₁₈-Alkylresten, C₇- bis C₂₀-Alkylarylresten, Resten -R⁵-O(C=O)-R⁶ und Resten -R⁵-NH(C=O)-R⁶, wobei R⁵ aus den C₂- bis C₄-Alkylenresten und R⁶ aus den C₁₀- bis C₁₈-Alkylresten ausgewählt ist, und in R¹, R², R³, R⁴ und R⁶ jeweils die Alkylanteile linear oder verzweigtkettig sein können.

2. Wasch-, Reinigungs- oder Haarbehandlungsmittel, enthaltend eine Mischung aus einem Rhamnolipid und einer Verbindung der allgemeinen Formel (I)
X^{a-} a N⁺(R¹R²R³R⁴) (I)
in der
X^{a-} ein Anion ist,
a für 1, 2 oder 3 steht und
R¹ ausgewählt ist aus R² und H, R² ausgewählt wird aus C₁- bis C₄-Alkylresten und C₂- bis C₄-Hydroxyalkylresten, R³ ausgewählt ist aus R⁴ und R², und R⁴ ausgewählt ist aus C₁₀- bis C₁₈-Alkylresten, C₇- bis C₂₀-Alkylarylresten, Resten -R⁵-O(C=O)-R⁶ und Resten -R⁵-NH(C=O)-R⁶, wobei R⁵ aus den C₂- bis C₄-Alkylenresten und R⁶ aus den C₁₀- bis C₁₈-Alkylresten ausgewählt ist, und in R¹, R², R³, R⁴ und R⁶ jeweils die Alkylanteile linear oder verzweigtkettig sein können.

3. Mittel nach Anspruch 2, **dadurch gekennzeichnet, dass** es 0,1 Gew.-% bis 40 Gew.-%, insbesondere 0,2 Gew.-% bis 30 Gew.-% und besonders bevorzugt 0,5 Gew.-% bis 15 Gew.-% der Mischung enthält.

4. Verfahren zum Waschen von Textilien, in dessen Verlauf eine Mischung aus einem Rhamnolipid und einer Verbindung der allgemeinen Formel (I)
X^{a-} a N⁺(R¹R²R³R⁴) (I)
in der
X^{a-} ein Anion ist,
a für 1, 2 oder 3 steht und
R¹ ausgewählt ist aus R² und H, R² ausgewählt wird aus C₁- bis C₄-Alkylresten und C₂- bis C₄-Hydroxyalkylresten, R³ ausgewählt ist aus R⁴ und R², und R⁴ ausgewählt ist aus C₁₀- bis C₁₈-Alkylresten, C₇- bis C₂₀-Alkylarylresten, Resten -R⁵-O(C=O)-R⁶ und Resten -R⁵-NH(C=O)-R⁶, wobei R⁵ aus den C₂- bis C₄-Alkylenresten und R⁶ aus den C₁₀- bis C₁₈-Alkylresten ausgewählt ist, und in R¹, R², R³, R⁴ und R⁶ jeweils die Alkylanteile linear oder verzweigtkettig sein können, in eine Waschflotte eingebracht wird, die ein reinigungsbedürftiges Textil enthält oder in die ein reinigungsbedürftiges Textil eingebracht wird.

5. Verfahren zur Reinigung von harten Oberflächen, in dessen Verlauf eine Mischung aus einem Rhamnolipid und einer Verbindung der allgemeinen Formel (I)
X^{a-} a N⁺(R¹R²R³R⁴) (I)
in der
X^{a-} ein Anion ist,
a für 1, 2 oder 3 steht und
R¹ ausgewählt ist aus R² und H, R² ausgewählt wird aus C₁- bis C₄-Alkylresten und C₂- bis C₄-Hydroxyalkylresten, R³ ausgewählt ist aus R⁴ und R², und R⁴ ausgewählt ist aus C₁₀- bis C₁₈-Alkylresten, C₇- bis C₂₀-Alkylarylresten, Resten -R⁵-O(C=O)-R⁶ und Resten -R⁵-NH(C=O)-R⁶, wobei R⁵ aus den C₂- bis C₄-Alkylenresten und R⁶ aus den C₁₀- bis C₁₈-Alkylresten ausgewählt ist, und in R¹, R², R³, R⁴ und R⁶ jeweils die Alkylanteile linear oder verzweigtkettig sein können, in eine Reinigungsflotte eingebracht wird, die mit der zu reinigenden harten Oberfläche in Kontakt gebracht wird.

6. Verfahren zur Haarbehandlung, in dessen Verlauf eine Mischung aus einem Rhamnolipid und einer Verbindung der allgemeinen Formel (I)
X^{a-} a N⁺(R¹R²R³R⁴) (I)
in der
X^{a-} ein Anion ist,
a für 1, 2 oder 3 steht und
R¹ ausgewählt ist aus R² und H, R² ausgewählt wird aus C₁- bis C₄-Alkylresten und C₂- bis C₄-Hydroxyalkylresten, R³ ausgewählt ist aus R⁴ und R², und R⁴ ausgewählt ist aus C₁₀- bis C₁₈-Alkylresten, C₇- bis C₂₀-Alkylarylresten, Resten -R⁵-O(C=O)-R⁶ und Resten -R⁵-NH(C=O)-R⁶, wobei R⁵ aus den C₂- bis C₄-Alkylenresten und R⁶ aus den C₁₀- bis C₁₈-Alkylresten ausgewählt ist, und in R¹, R², R³, R⁴ und R⁶ jeweils die Alkylanteile linear oder verzweigtkettig sein können, auf insbesondere nasse Haare aufgebracht wird.

7. Mischung gemäß Anspruch 1, Mittel gemäß Anspruch 2 oder 3, oder Verfahren gemäß einem der Ansprüche 4 bis 6, **dadurch gekennzeichnet, dass** in den Kationen N⁺(R¹R²R³R⁴) der Verbindungen der allgemeinen Formel (I) zwei bis drei, insbesondere drei der vier Reste eine C-Kettenlänge von höchstens 4, insbesondere von 1 aufweisen und der übrige Rest beziehungsweise die anderen beiden Reste eine Kohlenstoffkette von C₁₀ bis C₁₈ besitzen.

8. Mischung gemäß Anspruch 1 oder 7, Mittel gemäß einem der Ansprüche 2, 3 oder 7, oder Verfahren gemäß einem der Ansprüche 4 bis 7, **dadurch gekennzeichnet, dass** das molare Verhältnis von Rhamnolipid : [Verbindung der allgemeinen Formel (I) : a] im Bereich von 100:1 bis 1:1, insbesondere von 10:1 bis 1:1 liegt.

9. Mischung gemäß einem der Ansprüche 1, 7 oder 8, Mittel gemäß einem der Ansprüche 2, 3, 7 oder 8, oder Verfahren gemäß einem der Ansprüche 4 bis 8, **dadurch gekennzeichnet, dass** der Rhamnolipid-Anteil in der Mischung zu mindestens 50 Gew.-%, insbesondere zu mindestens 90 Gew.-% und gegebenenfalls zu 100 Gew.-% aus Di-Rhamnolipid besteht.
